# EUROPÄISCHE PATENTSCHRIFT

(11) **EP 0 778 256 B1**
(45) Veröffentlichungstag und Bekanntmachung des Hinweises auf die Patenterteilung: **27.10.1999**
(21) Anmeldenummer: 96118815.8
(22) Anmeldetag: 23.11.1996
(51) Int. Cl.: C07C 59/265

(54) **Verfahren zur Herstellung einer organischen Säure**
Process for producing an organic acid
Procédé de production d'acide organique

(30) Priorität: 05.12.1995 DE 19545303
(43) Veröffentlichungstag der Anmeldung: 11.06.1997
(73) Patentinhaber: Metallgesellschaft Aktiengesellschaft, 60325 Frankfurt am Main (DE)
(72) Erfinder: Ahlers, Bernd, 60388 Frankfurt am Main (DE); Bönsch, Rudolf, Dr., 55299 Nackenheim (DE); Eichelsbacher, Michael, 55130 Mainz (DE); Kuhn, Jürgen, 65929 Frankfurt am Main (DE); Sander, Ulrich, Dr., 61381 Friedrichsdorf (DE); Pendl, Jiri, 32322 Plzen (CZ); Hotek, Frantisek, 43985 Petrohrad (CZ); Cerny, Vaclav, 33151 Kaznejov (CZ)

(56) Entgegenhaltungen:
- EP-A- 0 151 470
- DE-C- 446 865
- GB-A- 1 494 414
- US-A- 2 253 061
- US-A- 4 851 573
- US-A- 5 032 686
- CHEMIE-INGENIEUR-TECHNIK, Bd. 62, Nr. 2, Februar 1990, WEINHEIM, Seiten 134-135, XP000141914 JÖRG VON EYSMONDT UND CHRISTIAN WANDREY: "INTEGRIERTE PRODUKTAUFARBEITUNG MITTELS ELEKTRODIALYSE BEI DER MIKROBIELLEN PRODUKTION ORGANISCHER SÄUREN"
- CHEMIE-INGENIEUR-TECHNIK, Bd. 62, Nr. 3, März 1990, WEINHEIM, Seite 214 XP000160083 MARTIN LOTZ UND MICHAEL CZYTKO: "KONTINUIERLICHE FERMENTATIVE HERSTELLUNG VON L-MILCHSÄURE UND IHRE AUFARBEITUNG DURCH ELEKTRODIALYSE"

## Beschreibung

Die Erfindung bezieht sich auf ein Verfahren zur Herstellung einer organischen Säure und/oder ihrer Salze aus einer Lösung, die durch Fermentation gewonnen wurde.

Bestimmte organische Säuren, wie Citronensäure, Milchsäure und Gluconsäure werden vorwiegend fermentativ hergestellt. Bei der Fermentation wird aus geeigneten, kohlehydrathaltigen Rohstoffen in Anwesenheit eines Mikroorganismus, beispielsweise Aspergillus niger bei der Herstellung von Citronensäure, eine Lösung der organischen Säure erzeugt. An die Fermentation schließen sich aufwendige Reinigungsverfahren dieser Säurelösung an, um die für ein verkäufliches Produkt erforderliche Reinheit zu erreichen. Auch eine Kristallisation der organischen Säure ist erst aus einer relativ reinen Säurelösung möglich, die einen geringen Anteil an nicht umgesetzten Kohlehydraten, Proteinen, Aminosäuren und Salzen enthält. Für eine besonders hohe Reinheit des Produkts, wie es beispielsweise für eine Anwendung im Lebensmittelbereich und insbesondere für ein Produkt nach der Spezifikation Pharmaqualität, zum Beispiel nach USP-Norm, beschrieben in der "Pharmacopeia of the United States", XXII Edition, Seite 315, gefordert wird, müssen auch die Kristallite eine sehr hohe Reinheit aufweisen. Dies wird nur durch aufwendige Reinigungsverfahren erreicht. Die Reinigung der Citronensäure wird zum Beispiel durch Fällen der Säure als Calciumsalz, Abtrennen des Calciumcitrats und Umsetzen des gebildeten Calciumcitrats mit Schwefelsäure durchgeführt. Dabei werden große Mengen an Schwefelsäure verbraucht und darüber hinaus fällt verunreinigtes Calciumsulfat sowie eine große Abwassermenge an. Auch bei Verwendung von relativ reinen kohlehydrathaltigen Rohstoffen für die Fermentation ist es bisher notwendig, aufwendige Reinigungsverfahren anzuwenden. Derartige kohlehydrathaltige Rohstoffe sind beispielsweise Glucose, Dextrose, Saccharose oder gereinigte und verflüssigte Stärke.

In der US-A 4,275,234 ist ein Verfahren beschrieben, bei dem die organische Säure aus einer wäßrigen Lösung durch ein wasserunlösliches Lösungsmittel extrahiert wird. Nach einem in der EP-A-O 167 957 offenbarten Verfahren wird die organische Säure an einem Ionenaustauscher adsorbiert und anschließend wieder desorbiert. In der DE-A 3 502 924 ist ebenfalls ein Verfahren beschrieben, bei dem anschließend an eine Membranfiltration eine Adsorption an einem nicht-ionogenen Adsorberharz durchgeführt wird. Allen zuvor genannten Verfahren ist gemeinsam, daß ein großer Verlust an organischer Säure bei der notwendigen Desorption auftritt und daß ein hoher Aufwand nötig ist, um diesen Verlust sinnvoll zu begrenzen. Darüber hinaus sind weitere Reinigungsschritte notwendig, um organische Säuren sehr hoher Reinheit und entsprechender Farbe zu erhalten.

In der EP-A-O 163 836 und in der EP-A-O 479 048 sind Verfahren zur Granulation von organischen Säuren beschrieben. Bei diesen Verfahren sind in der zu granulierenden Lösung noch Restgehalte an nicht umgesetztem kohlehydrathaltigen Rohstoff von 2 bis 30 Gew.-%, bezogen auf die durch Fermentation erzeugte Säure, enthalten. Die Verfahren zur Granulierung dienen dabei nicht der weiteren Reinigung der Säure, sondern sie dienen vielmehr der Überführung der flüssigen Säure in einen für Transportzwecke günstigen, festen granulierten Zustand.

Es ist die Aufgabe der vorliegenden Erfindung, ein effektives und kostengünstiges Verfahren bereitzustellen, mit dem aus einer durch Fermentation gewonnenen Lösung eine organische Säure und/oder ihre Salze hergestellt werden kann, die relativ hohen Reinheitsanforderungen bis hin zur Pharmaqualität genügt. Ferner sollen dabei die Nachteile des Reinigungverfahrens mittels Fällung der Säure als Calciumsalz vermieden werden. Insbesondere sollen keine großen Mengen an Abfallstoffen, beispielsweise verunreinigtes Calciumsulfat und Abwasser, anfallen.

Die Aufgabe wird durch ein Verfahren gelöst, bei dem ein reiner, kohlehydrathaltiger Rohstoff für die Substratherstellung verwendet wird, bei dem die mittels Fermentation hergestellte säurehaltige Lösung einer Zellabtrennung zugeführt wird, bei dem das säurehaltige Permeat aus der Zellabtrennung einer Proteinfällung zugeführt wird, wobei es mit einem siliciumhaltigen Fällungsmittel bei Temperaturen zwischen 2 bis 70 °C versetzt wird, bei dem die so erhaltene Lösung einer Proteinabtrennung zugeführt wird und bei dem das säurehaltige Permeat aus der Proteinabtrennung konzentriert und anschließend einer ein- oder mehrstufigen Kristallisation oder einer Granulation zugeführt wird.

Unter "reinem", kohlehydrathaltigen Rohstoff ist hier zu verstehen, daß der Reinheitsgrad beispielsweise für Zucker größer ca. 95 % ist. Als kohlehydrathaltige Rohstoffe können beispielsweise Glucose, Dextrose, Saccharose oder gereinigte und verflüssigte Stärke verwendet werden. Mit Hilfe der Zellabtrennung werden vor allem höhermolekulare Verunreinigungen, insbesondere Biomasse, wie Mycel, und koagulierte Proteine, abgetrennt. Die Zellabtrennung kann zum Beispiel durch Filtration oder Zentrifugation erfolgen. Mit dem siliciumhaltigen Fällungsmittel werden insbesondere gelöste Proteine als Verunreinigungen ausgefällt, wobei eine Temperatur von 2 bis 70 °C eine wirksame Ausfällung und eine sichere Verfahrensweise gewährleistet. Die Proteinabtrennung erfolgt zum Beispiel durch Filtration oder Zentrifugation. Es werden ausgefällte Verunreinigungen und restliche, höhermolekulare Verunreinigungen abgetrennt. Die Lösung wird beispielsweise durch ein- oder mehrstufiges Eindampfen konzentriert. Aus dieser aufkonzentrierten Säurelösung ist eine direkte Kristallisation des Endprodukts möglich. Die Kristallisation wird zum Beispiel als Verdampfungskristallisation oder Kühlkristallisation durchgeführt, was kontinuierlich oder batchweise erfolgen kann. Als organische Säuren können mit Hilfe des erfindungsgemäßen Verfahrens beispielsweise Citronensäure, Milchsäure und Gluconsäure hergestellt werden. Die so hergestellten Säuren weisen eine überraschend hohe Reinheit bis hin zu den Reinheitsanforderungen der Lebensmittelqualität auf. Ein besonderer Vorteil des erfindungsgemäßen Verfahrens ist es, daß bei dieser Fällung mit einem siliciumhaltigen Fällungsmittel nur Verunreinigungen ausgefällt werden. Im Gegensatz dazu wird bei den Fällungsverfahren des Standes der Technik die gesamte Säurelösung, beispielsweise als Calciumsalz, gefällt und es entstehen sehr große Mengen an Abfallstoffen, beispielsweise Calciumsulfat. Darüber hinaus entstehen bei den Verfahren mittels Fällung als Calciumsalz auch sehr große Mengen an Abwasser, die beim erfindungsgemäßen Verfahren nicht anfallen. Daher ist das erfindungsgemäße Verfahren aus ökonomischer und ökologischer Sicht den Verfahren nach dem Stand der Technik deutlich überlegen.

In weiterer Ausgestaltung der Erfindung ist es vorgesehen, daß als reiner, kohlehydrathaltiger Rohstoff für die Substratherstellung reine Glucose, Saccharose oder reine, enzymatisch verflüssigte Stärke der Raffinatsstufe 1 mit einem Anteil an höheren Zuckern kleiner 5 % verwendet wird. Dabei hat es sich als besonders vorteilhaft erwiesen, wenn die Glucose und Saccharose nur einen Anteil an höheren Zuckern kleiner 1 bis 2 % aufweist.

In weiterer Ausgestaltung der Erfindung ist es vorgesehen, daß der reine, kohlehydrathaltige Rohstoff für die Substratherstellung vor seiner Fermentation einem Kationenaustausch zugeführt wird. Durch diese Verfahrensführung werden Metallkationen, wie Eisen und Magnesium und insbesondere Mangan, abgetrennt, die den Prozeß der Säureherstellung sonst stören würden.

In weiterer Ausgestaltung der Erfindung ist es vorgesehen, daß die Zellabtrennng mit Hilfe einer Filtration durchgeführt wird. Mit Hilfe dieses technisch relativ einfach zu realisierenden und damit kostengünstigen Verfahrens kann der Gehalt an höhermolekularen Verunreinigungen in der Lösung deutlich gemindert werden. Es kann beispielsweise ein Bandfilter, Vakuum-Trommelfilter oder Membranfilter verwendet werden. Die Membranfiltration kann bei Lösungen mit einer Temperatur von kleiner ca. 50 °C verwendet werden. Durch diese Trennung werden die höhermolekularen Verunreinigungen bis auf einen Gehalt kleiner ca. 1 g/l abgetrennt.

In weiterer Ausgestaltung der Erfindung ist es vorgesehen, daß die Zellabtrennng mit Hilfe einer Membranfiltration mit einem Filter einer Porenweite von 0,1 bis 5 µm durchgeführt wird. Der Vorteil dieser Porenweite liegt in einer überraschend hohen Permeatleistung des Filters.

In weiterer Ausgestaltung der Erfindung ist es vorgesehen, daß das Permeat aus der Zellabtrennung vor der Proteinfällung für eine Zeitdauer von 0,5 bis 10 min. auf eine Temperatur größer 70 °C erhitzt wird. Durch diese Erhitzung werden die zu fällenden Proteine zumindest teilweise koaguliert und aktive Proteasen des Pilzmycels desaktiviert.

In weiterer Ausgestaltung der Erfindung ist es vorgesehen, daß das Sediment aus der Proteinfällung nach einer Absetzzeit von 30 bis 300 min. in die säurehaltige Lösung vor die Zellabtrennung zurückgeführt wird und daß der Überstand aus der Proteinfällung der Proteinabtrennung zugeführt wird. Durch die Rückführung des Sediments aus der Proteinfällung wird die anschließende Zellabtrennung verbessert.

In weiterer Ausgestaltung der Erfindung ist es vorgesehen, daß die Proteinabtrennung mit Hilfe einer Membranfiltration durchgeführt wird. Die Membranfiltration kann beispielsweise als cross-flow-Verfahren mit Hilfe von Membranen aus einem keramischen Material durchgeführt werden.

In weiterer Ausgestaltung der Erfindung ist es vorgesehen, daß die Proteinabtrennung mit Hilfe einer Membranfiltration durchgeführt wird, die aus einer Kombination einer Mikrofiltration mit einem Filter einer Porenweite von 0,05 bis 5 µm und einer Ultrafiltration mit einer Partikelausschlußgrenze bis unter 5000 Dalton besteht. Durch diese Verfahrensführung werden in vorteilhafter Weise die höhermolekularen Verunreinigungen bis auf einen Gehalt von kleiner ca. 2 mg/l abgetrennt.

In weiterer Ausgestaltung der Erfindung ist es vorgesehen, daß die Proteinabtrennung mit Hilfe einer Membranfiltration durchgeführt wird, die aus einer Kombination einer Mikrofiltration mit einem Filter einer Porenweite von 0,05 bis 5 µm, einer Ultrafiltration mit einer Partikelausschlußgrenze bis unter 2000 Dalton und einer Nanofiltration mit einer Partikelausschlußgrenze bis unter 200 Dalton besteht. Durch diese Verfahrensführung werden in vorteilhafter Weise die höhermolekularen Verunreinigungen bis auf einen Gehalt von kleiner ca. 1 mg/l abgetrennt.

In weiterer Ausgestaltung der Erfindung ist es vorgesehen, daß das Retentat aus der Proteinabtrennung in die säurehaltige Lösung vor die Zellabtrennung zurückgeführt wird oder mit vollentsalztem Wasser ausgewaschen wird und die so eluierte Citronensäure einem Ionenaustauscher, einer Membran-Elektrodialyse oder einer Fällung als Calciumsalz zugeführt wird und anschließend in die säurehalige Lösung vor die Zellabtrennung zurückgeführt oder einer weiteren Verwertung zugeführt wird. Durch diese Verfahrensführung kann in vorteilhafter Weise die übrige Säure in hoher Konzentration abgetrennt werden. Das dadurch gewonnene Konzentrat der Säure kann für eine weitere Verwertung kristallisiert oder granuliert werden. Das citronensäurefreie bzw. citronensäurearme Retentat der Proteinabtrennung kann mit dem gewaschenen Mycel aus der Zellabtrennung vereinigt werden.

In weiterer Ausgestaltung der Erfindung ist es vorgesehen, daß das Permeat aus der Proteinabtrennung einem Ionenaustausch und/oder einer Entfärbung zugeführt wird. Als Ionenaustauscher können zum Beispiel Kationenaustauscher und schwach basische Anionenaustauscher auf Basis eines Polymers oder Gels verwendet werden. Dadurch werden in vorteilhafter Weise restliche, insbesondere niedermolekulare Salze und Aminosäuren abgetrennt. Der Restgehalt an Verunreinigungen liegt dadurch im Bereich von 0,2 bis 2 mg/l an freiem Aminosäuren-Stickstoff. Die Entfärbung kann in vorteilhafter Weise durch eine Behandlung mit Aktivkohle erfolgen, wobei diese Behandlung beispielsweise vor der Konzentrierung der Lösung durchgeführt wird. Die Aktivkohlebehandlung kann beispielsweise in einem Festbett erfolgen.

In weiterer Ausgestaltung der Erfindung ist es vorgesehen, daß der Ionenaustauscher aus einem stark sauren Kationenaustauscher und/oder einem schwach basischen Anionenaustauscher besteht und daß vom Anionenaustauscher mit einer 5 bis 10 %-igen Salzsäure-Lösung zuerst Citronensäure eluiert wird und anschließend der Anionenaustauscher mit einer 3 bis 7 %-igen Natronlauge-Lösung regeneriert wird oder daß der Anionenaustauscher mit einer Ammoniak-Lösung regeneriert wird. Auf dem Kationenaustauscher werden neben den Kationen ein großer Teil der in der Lösung enthaltenen Aminosäuren zurückgehalten und der Anionenaustauscher hält die Anionen und einen weiteren Teil an Aminosäuren zurück. Die Natronlauge zur Regeneration wird vorzugsweise in einer Konzentration von ca. 5% verwendet. Eine 2 bis 3 %-ige Ammoniak-Lösung hat sich als besonders geeignet für die Regeneration des Anionenaustauschers erwiesen.

In weiterer Ausgestaltung der Erfindung ist es vorgesehen, daß die Kristallisation in drei Stufen erfolgt, wobei die Kristallite aus der ersten Kristallisationsstufe in vollentsalztem Wasser gelöst werden und einer zweiten Kristallisationsstufe zugeführt werden, wobei die Mutterlauge aus der zweiten Kristallisationsstufe der dritten Kristallisationsstufe zugeführt wird, wobei die Kristallite aus der dritten Kristallisationsstufe in vollentsalztem Wasser gelöst werden und in die zweite Kristallisationsstufe zurückgeführt werden und wobei die Mutterlauge der dritten Kristallisationsstufe einer weiteren Verwertung zugeführt wird. Die Kristallite aus der zweiten Kristallisationsstufe können nach einer Trocknung bzw. Sprühtrocknung als Kristalle bzw. Säure-Granulate in den Handel gebracht werden. Diese Säure besitzt eine Reinheit bis hin zur Pharmaqualität.

In weiterer Ausgestaltung der Erfindung ist es vorgesehen, daß die Mutterlauge aus der Kristallisation bzw. dritten Kristallisationsstufe der Säure neutralisiert wird und das Salz der organischen Säure einer ein- oder mehrstufigen Salzkristallisation zugeführt wird. Die Neutralisierung kann beispielsweise mit Natronlauge erfolgen, wodurch ein Natriumsalz der organischen Säure gebildet wird.

In weiterer Ausgestaltung der Erfindung ist es vorgesehen, daß die Salzkristallisation in drei Stufen erfolgt, wobei die Mutterlauge aus der ersten Salzkristallisationsstufe einer zweiten Salzkristallisationsstufe zugeführt wird, wobei die Kristallite der zweiten Salzkristallisationsstufe mit denen der ersten Salzkristallisationsstufe vereinigt werden, wobei die vereinigten Kristallite aus der ersten und zweiten Salzkristallisationsstufe in vollentsalztem Wasser gelöst und einer dritten Salzkristallisationsstufe zugeführt werden und wobei die Mutterlauge aus der dritten Salzkristallisationsstufe mit der Mutterlauge aus der zweiten Salzkristallisationsstufe vereinigt wird und einer weiteren Verwendung zugeführt wird. Die Kristallite aus der dritten Salzkristallisationsstufe können nach einer Trocknung bzw. Sprühtrocknung als Kristalle bzw. Säure-Granulate in den Handel gebracht werden. Diese Säure besitzt eine Reinheit bis hin zur Pharmaqualität.

In weiterer Ausgestaltung der Erfindung ist es vorgesehen, daß die Mutterlauge aus der dritten Kristallisationsstufe oder die Mutterlauge aus der Salzkristallisation bzw. die vereinigte Mutterlauge aus der zweiten und dritten Salzkristallisationsstufe einem Ionenaustausch und/oder einer Entfärbung zugeführt wird. Für den Ionenaustausch kann zum Beispiel ein Kationenaustauscher oder eine Kombination aus einem Kationen- und Anionenaustauscher verwendet werden. Dadurch werden in vorteilhafter Weise niedermolekulare Salze und Aminosäuren abgetrennt. Die Entfärbung kann in vorteilhafter Weise durch eine Behandlung mit Aktivkohle, beispielsweise in einem Festbett, erfolgen.

In weiterer Ausgestaltung der Erfindung ist es vorgesehen, daß die Kristallite aus der Kristallisation bzw. zweiten Kristallisationsstufe und/oder der Salzkristallisation bzw. dritten Salzkristallisationsstufe in einer Flüssigkeit suspendiert und anschließend granuliert werden. Die Säurekristallite können zum Beispiel in vollentsalztem Wasser suspendiert werden und mit Hilfe der Sprühgranulation in ein festes Produkt überführt werden. Durch diese Verfahrensführung können in vorteilhafter Weise Säuregranulate mit verbesserten Feststoffeigenschaften, verglichen mit den ursprünglichen Kristalliten, hergestellt werden.

In weiterer Ausgestaltung der Erfindung ist es vorgesehen, daß die Mutterlauge aus der Kristallisation bzw. ersten Kristallisationsstufe und/oder der Salzkristallisation bzw. dritten Salzkristallisationsstufe einem Ionenaustauscher, einer Membran-Elektrodialyse oder einer Fällung als Calciumsalz zugeführt wird und anschließend in das Permeat aus der Zellabtrennung zurückgeführt oder einer weiteren Verwertung zugeführt wird. Durch diese Verfahrensführung kann in vorteilhafter Weise die übrige Säure in hoher Konzentration von Verunreinigungen abgetrennt werden. Das dadurch gewonnene Konzentrat der Säure kann für eine weitere Verwertung kristallisiert oder granuliert werden. Beispielsweise kann die Mutterlauge in vorteilhafter Weise mit Hilfe der Sprühgranulation in ein festes Produkt überführt werden. Das so erhaltene Säuregranulat kann als festes Handelsprodukt beispielsweise in der Verfahrenstechnik aber auch in der Lebensmitteltechnik eine Anwendung finden.

In weiterer Ausgestaltung der Erfindung ist es vorgesehen, daß als siliciumhaltiges Fällungsmittel eine 25 bis 35 %-ige SiO₂-Lösung mit SiO₂ einer spezifischen Oberfläche größer 100 m²/g verwendet wird. In der 25 bis 35 %-igen SiO₂-Lösung ist das SiO₂ vorzugsweise kolloidal gelöst. Es hat sich gezeigt, daß als Fällungsmittel beispielsweise eine 30 %-ige SiO₂-Lösung mit SiO₂ einer spezifischen Oberfläche von mindestens 100 m²/g sehr gut geeignet ist, wobei das SiO₂ vorzugsweise eine spezifische Oberfläche von mindestens 500 m²/g haben sollte.

In weiterer Ausgestaltung der Erfindung ist es vorgesehen, das erfindungsgemäße Verfahren zur Herstellung von Citronensäure zu verwenden. Die Herstellung von Citronensäure mit dem erfindungsgemäßen Verfahren ist bevorzugt, da durch die Proteinfällung mit dem siliciumhaltigen Fällungsmittel, bei einem pH-Wert von ca. 1,5 bis 2,0, eine relativ große Menge an Proteinen und restlichen, höhermolekularen Verunreinigungen ausgefällt wird. Eine Herstellung einer relativ reinen Citronensäure ohne eine Fällung dieser Säure als Calciumsalz in vorteilhafter Weise ist so möglich.

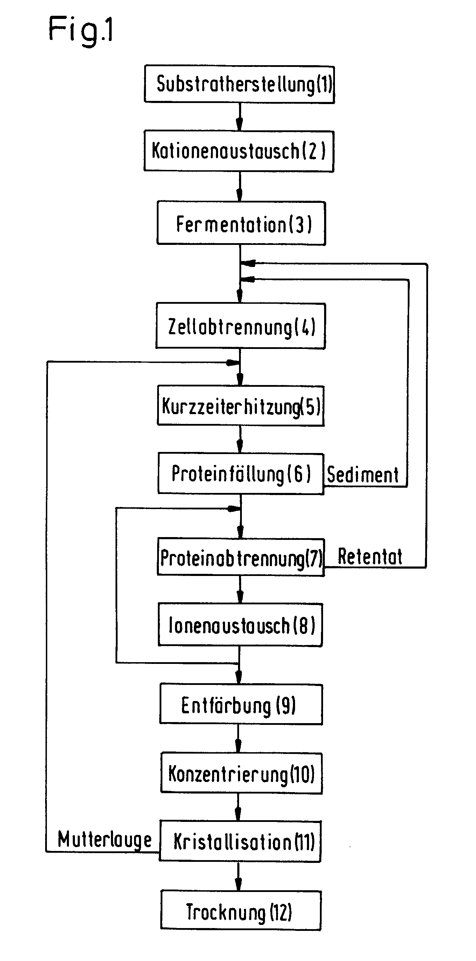

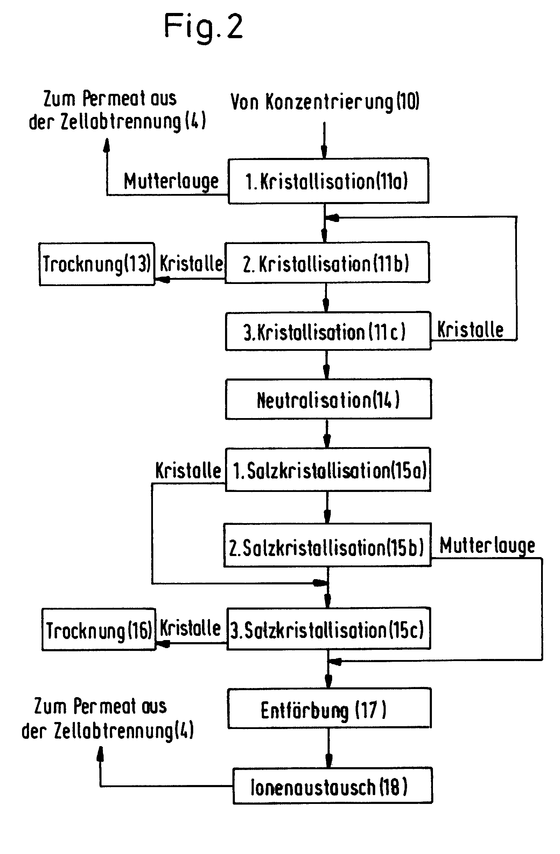

Die Erfindung wird nun anhand von Ausführungsbeispielen und von zwei Verfahrensfließbildern (Fig.1 und Fig. 2) näher erläutert.

Fig. 1 zeigt den Ablauf des erfindungsgemäßen Verfahrens am Beispiel der Herstellung von Citronensäure in Lebensmittelqualität.

Fig. 2 zeigt den Ablauf des erfindungsgemäßen Verfahrens am Beispiel der Herstellung von Citronensäure in Pharmaqualität.

### Beispiel 1: Herstellung von Citronensäure aus einer fermentativ gewonnenen Citronensäure-Lösung als kristallines oder granuliertes Handelsprodukt in Lebensmittelqualität (siehe Fig. 1).

Zunächst wird das Substrat hergestellt (1). Dazu wird hochreine, enzymatisch verflüssigte Stärke oder Glucosesirup verdünnt oder kristalline Glucose oder Sacharose der Raffinatsstufe 1 in entsprechender Konzentration aufgelöst. Die so hergestellte Zucker- oder Stärkelösung wird über einen Kationenaustauscher (2) geführt und anschließend einer kontinuierlichen Pasteurisierung unterzogen. Die für die Fermentation notwendigen Salze werden in einem separaten Rührkessel angesetzt. Das Medium aus der Substratherstellung (1) wird einer Fermentation (3) zugeführt. Dort wird das Medium in einem Fermenter mit Sporen des Pilzes Aspergillus niger beimpft. Nach der Fermentation (3) wird die Lösung einer Zellabtrennung (4) zugeführt. Dort werden die Zellen zum Beispiel mit Hilfe eines Vakuumbandfilters aus der Fermentationslösung abgetrennt. Die Herstellung der Citronensäure-Lösung erfolgt im Batchbetrieb mit einem Batchvolumen von mindestens 50 m³, vorzugsweise 150 bis 200 m³, und einer Citronensäuremenge von 180 bis 200 kg Citronensaure-Monohydrat/m³ bzw. 27 bis 40 t Citronensäure-Monohydrat/Batch. Das Permeat aus der Zellabtrennung (4) enthält ca. 10 bis 25 % Citronensäure in gelöster Form und neben anderen gelösten organischen und anorganischen Bestandteilen noch eine geringe Menge an ungelöster restlicher Biomasse. Das säurehaltige Permeat wird für eine Zeitdauer von 2 bis 3 min. auf eine Temperatur von ca. 75 °C erhitzt (5) und anschließend einer Proteinfällung (6) zugeführt. Hier werden im Batchbetrieb kolloidal gelöste Proteine und andere organische Substanzen durch Zugabe von 1 bis 10 l/m³ einer 30 %-igen SiO₂-Lösung mit SiO₂ einer spezifischen Oberfläche von mindestens 500 m²/g gefällt. Die Temperatur bei der Proteinfällung (6) beträgt ca. 50 °C und der pH-Wert der Lösung liegt im Bereich von 1,5 bis 2,0. Nach einer relativ kurzen Absetzzeit von 1 bis 2 Stunden wird das Sediment zur Fermentationslösung vor der Zellabtrennung (4) zurückgegeben und der Überstand einer Proteinabtrennung (7) mit Hilfe einer Membranfiltration zugeführt. Die Membranfiltration wird kontinuierlich betrieben mit einem Volumenstrom, der abhängig ist von der jeweiligen Bauart und Größe der Filtrationsanlage. Er kann beispielsweise in einem Bereich von 5 bis 15 m³/h liegen. Die Membranfiltration wird mit Hilfe eines Verfahrens durchgeführt, welches aus einer Kombination einer Mikrofiltration mit einem Filter einer Porenweite von 0,05 bis 5 µm und einer Ultrafiltration mit einer Partikelausschlußgrenze bis unter 5000 Dalton, vorzugsweise 2000 Dalton, besteht. Zusätzlich ist eine Nanofiltration mit einer Partikelausschlußgrenze bis unter 200 Dalton möglich. Während der Proteinabtrennung (7) wird das Retentat aufkonzentriert und zur Fermentationslösung vor der Zellabtrennung (4) gegeben oder aus dem Retentat der Proteinabtrennung (7) wird anhaftende Citronensäure mittels entsalztem Wasser eluiert und in den Prozeß zurückgeführt, während das dann fast vollständig citronensäurefreie Retentat aus dem Prozeß ausgeschleust wird. Das Retentat beinhaltet Zellen als ungelöste Substanzen, die nicht mit dem Vakuumbandfilter abgeschieden worden sind, sowie gefällte Proteine und andere organische Bestandteile mit einer Teilchengröße von mindestens 5000 bis 2000 bzw. 200 Dalton. Das Permeat, eine klare Lösung, enthält gelöste Citronensäure sowie gelöste organische und anorganische Bestandteile. Zur weiteren Verarbeitung wird das Permeat aus der Proteinabtrennung (7) kontinuierlich über einen Ionenaustauscher (8) geführt. Der Ionenaustauscher (8) besteht zum Beispiel aus zwei Stufen mit einem stark sauren Kationenaustauscher und einem schwach basischen Anionenaustauscher. Auf dem Kationenaustauscher werden neben den Kationen ein großer Teil der in der Lösung enthaltenen Aminosäuren zurückgehalten. Der Anionenaustauscher hält die Anionen und einen weiteren Teil an Aminosäuren zurück. Ein Durchbruch von Substanzen wird mit einer Leitfähigkeitsmeßsonde oder einer pH-Sonde gemessen. Bei Bedarf wird auf ein anderes Ionenaustauscherpaar umgeschaltet, so daß eine relativ sichere Abtrennung von störenden Kationen, Anionen und Aminosäuren gewährleistet werden kann. Vom Anionenaustauscher wird mit einer 5 bis 10 %-igen Salzsäure-Lösung Citronensäure eluiert und er wird anschließend mit einer ca. 5 %-igen Natronlauge-Lösung regeneriert oder der Anionenaustauscher wird mit einer Ammoniak-Lösung, vorzugsweise 2 bis 3 %-iger Ammoniak-Lösung, regeneriert. Bei der Regeneration mit einer Ammoniak-Lösung bildet sich Ammonium-Citrat, welches durch Erhitzung zersetzt wird. Dadurch wird wieder Citronensäure gebildet. Diese Citronensäure kann dem Prozeß wieder zugeführt werden. Sie kann entweder in die Lösung nach der Zellabtrennung (4), oder aber in die Lösung vor der Ultrafiltration der Proteinabtrennung (7) zurückgeführt werden. Der Ammoniak kann durch Wäsche als eine bis zu 20 % reine Stammlösung zurückgewonnen werden. Es ist ebenso möglich, den regenerierten Ionenaustauscher zu einer Reinigung der Mutterlauge, die im Verlauf des weiteren Verfahrens gebildet wird, zu verwenden. Dies ist insbesondere bei relativ hohen Konzentrationen an Restzucker und freien Aminosäuren in der Mutterlauge besonders vorteilhaft. Diese relativ hohen Konzentrationen können zum Beispiel dann entstehen, wenn die Mutterlauge in einem Kreislauf geführt wird. Die so aufgearbeitete Citronensäurelösung enthält eine bestimmte Menge an färbenden Substanzen. Diese werden in einer Entfärbung (9) in einem Aktivkohlefestbett kontinuierlichen aus der Lösung entfernt. Die entfärbte Lösung wird einer Konzentrierung (10) zugeführt, wobei die Säurelösung mit Hilfe eines mehrstufigen Verfahrens bis auf eine Konzentration von 60 bis 70 %, vorzugsweise ca. 67 %, kontinuierlich eingedampft wird. Die aufkonzentrierte Citronensäure wird einer Kristallisation (11) zugeführt oder aufkonzentriert und granuliert. Die Mutterlauge der Kristallisation (11) wird in das Permeat der Zellabtrennung (4) zurückgeführt. Zuvor kann die Mutterlauge mit Hilfe eines Ionenaustauschers, einer Elektrodialyse oder einer Fällung der Säure als Citrat aufgearbeitet werden. Die in dieser ersten Kristallisation erzeugten Citronensäure-Kristallite weisen bereits eine Reinheit entsprechend der Lebensmittelqualität auf. Diese Kristallite können nach erfolgter Trocknung (12) als Endprodukt in den Handel gebracht werden.

### Beispiel 2: Herstellung von Citronensäure aus einer fermentativ gewonnenen Citronensäure-Lösung als kristallines oder granuliertes Handelsprodukt in Pharmaqualität (siehe Fig. 2).

Die aus dem Verfahren gemäß Beispiel 1 gewonnenen Kristallite aus der ersten Kristallisationsstufe (11a) werden in vollentsalztem Wasser gelöst und einer zweiten Kristallisationsstufe (11 b) unterzogen. Nach einer Trocknung (13) können sie in den Handel gebracht werden. Diese Kristallite haben bereits Pharmaqualität nach USP- bzw. BP-Norm. Aus der Mutterlauge der zweiten Kristallisationsstufe (11b) werden in einer dritten Kristallisationsstufe (11c) weitere Kristalle gewonnen, die in vollentsalztem Wasser gelöst und in die zweite Kristallisationsstufe (11b) zurückgeführt werden. Die Mutterlauge der dritten Kristallisationsstufe (11c), die neben Citronensäure noch die restlichen im Prozeß verbliebenen Verunreinigungen in konzentrierter Form enthält, wird zur Gewinnung von Natrium-Citrat weiterverwendet, granuliert oder aufgearbeitet und der Proteinfällung (6) wieder zugeführt. Die Aufarbeitung kann mit Hilfe eines Ionenaustauschers, einer Elektrodialyse oder einer Fällung der Säure als Calcium-Citrat erfolgen. Die Kristallisation erfolgt kontinuierlich oder im Batchbetrieb.

### Beispiel 3: Herstellung von Natrium-Citrat aus der Mutterlauge einer Kristallisation von fermentativ gewonnener Citronensäure-Lösung als kristallines oder granuliertes Handelsprodukt in Pharmaqualität (siehe Fig. 2).

Die Mutterlauge aus der dritten Kristallisationsstufe (11c) gemäß dem Beispiel 2 wird einer Neutralisation zugeführt (14). Hier wird die säurehaltige Mutterlauge durch Zugabe von Natronlauge neutralisiert und einer ersten Salzkristallisationsstufe (15a) unterworfen, wobei Kristallite aus Natrium-Citrat entstehen. Aus der dabei anfallenden Mutterlauge werden in einer zweiten Salzkristallisationsstufe (15b) weitere Kristallite gewonnen, die mit denen der ersten Salzkristallisationsstufe (15a) vereinigt werden. Die vereinigten Kristallite werden in vollentsalztem Wasser gelöst und einer dritten Salzkristallisationsstufe (15c) unterworfen. Die Mutterlauge aus der dritten Salzkristallisationsstufe (15c) wird mit der Mutterlauge aus der zweiten Salzkristallisationsstufe (15b) vereinigt. Die Kristallite aus der dritten Salzkristallisationsstufe (15c) besitzen Pharmaqualität nach USP- bzw. BP-Norm. Sie werden einer Trocknung (16) oder Sprühtrocknung zugeführt und als kristallines oder granuliertes Handelsprodukt verwendet. Die Gewinnung des Natrium-Citrats wird kontinuierlich betrieben. Die Mutterlaugen aus der zweiten (15b) und dritten Salzkristallisationsstufe (15c) enthalten neben Citronensäure noch restliche Verunreinigungen aus dem Prozeß. Aus diesen Mutterlaugen wird die noch verbliebene Citronensäure zurückgewonnen und zum Aufarbeitungsprozeß zurückgegeben. Zur Rückgewinnung der noch verbliebenen Citronensäure werden die Mutterlaugen einer Entfärbung (17) auf einem Aktivkohlefestbett und anschließend einem Kationaustausch (18) zugeführt, um freie Citronensäure zu erhalten. Die Aufarbeitung kann auch durch einen weiteren Ionenaustausch, eine Membran-Elektrodialyse oder eine Fällung der Säure als Calcium-Citrat erfolgen. Diese Citronensäurelösung wird zum Permeat aus der Zellabtrennung (4) in den Prozeß zurückgeführt. Die Entfärbung (17) und der Kationenaustausch (18) werden kontinuierlich betrieben.

Durch das in Beispiel 3 beschriebene Verfahren wird eine Gesamtausbeute an Citronensäure von insgesamt ca. 95 % erreicht, wobei ca. 70 bis 90 % in der ersten Verfahrensstufe gemäß Beispiel 2 und weitere ca. 20 bis 30 % durch die zweite Verfahrensstufe gemäß Beispiel 3 erhalten werden.

## Patentansprüche

1. Verfahren zur Herstellung einer organischen Säure und/oder ihrer Salze aus einer Lösung, die durch Fermentation (3) gewonnen wurde, bei dem ein reiner, kohlehydrathaltiger Rohstoff für die Substratherstellung (1) verwendet wird, bei dem die mittels Fermentation (3) hergestellte säurehaltige Lösung einer Zellabtrennung (4) zugeführt wird, bei dem das säurehaltige Permeat aus der Zellabtrennung (4) einer Proteinfällung (6) zugeführt wird, wobei es mit einem siliciumhaltigen Fällungsmittel bei Temperaturen zwischen 2 bis 70 °C versetzt wird, bei dem die so erhaltene Lösung einer Proteinabtrennung (7) zugeführt wird und bei dem das säurehaltige Permeat aus der Proteinabtrennung (7) konzentriert (10) und anschließend einer ein- oder mehrstufigen Kristallisation (11) oder einer Granulation zugeführt wird.

2. Verfahren nach Anspruch 1, bei dem als reiner, kohlehydrathaltiger Rohstoff für die Substratherstellung (1) reine Glucose, Saccharose oder reine, enzymatisch verflüssigte Stärke der Raffinatsstufe 1 mit einem Anteil an höheren Zuckern kleiner 5 % verwendet wird.

3. Verfahren nach Anspruch 1 oder 2, bei dem der reine, kohlenhydrathaltige Rohstoff für die Substratherstellung (1) vor seiner Fermentation (3) einem Kationenaustausch (2) zugeführt wird.

4. Verfahren nach einem der Ansprüche 1 bis 3, bei dem die Zellabtrennng (4) mit Hilfe einer Filtration durchgeführt wird.

5. Verfahren nach Anspruch 4, bei dem die Zellabtrennung (4) mit Hilfe einer Mikrofiltration mit einem Filter einer Porenweite von 0,1 bis 5 µm durchgeführt wird.

6. Verfahren nach einem der Ansprüche 1 bis 5, bei dem das Permeat aus der Zellabtrennung (4) vor der Proteinfällung (6) für eine Zeitdauer von 0,5 bis 10 min. auf eine Temperatur größer 70 °C erhitzt wird (5).

7. Verfahren nach einem der Ansprüche 1 bis 6, bei dem das Sediment aus der Proteinfällung (6) nach einer Absetzzeit von 30 bis 300 min. in die säurehaltige Lösung vor die Zellabtrennung (4) zurückgeführt wird und bei dem der Überstand aus der Proteinfällung (6) der Proteinabtrennung (7) zugeführt wird.

8. Verfahren nach einem der Ansprüche 1 bis 7, bei dem die Proteinabtrennung (7) mit Hilfe einer Membranfiltration durchgeführt wird.

9. Verfahren nach Anspruch 8, bei dem die Proteinabtrennung (7) mit Hilfe einer Membranfiltration durchgeführt wird, die aus einer Kombination einer Mikrofiltration mit einem Filter einer Porenweite von 0,05 bis 5 µm und einer Ultrafiltration mit einer Partikelausschlußgrenze bis unter 5000 Dalton besteht.

10. Verfahren nach Anspruch 8, bei dem die Proteinabtrennung (7) mit Hilfe einer Membranfiltration durchgeführt wird, die aus einer Kombination einer Mikrofiltration mit einem Filter einer Porenweite von 0,05 bis 5 µm, einer Ultrafiltration mit einer Partikelausschlußgrenze bis unter 2000 Dalton und einer Nanofiltration mit einer Partikelausschlußgrenze bis unter 200 Dalton besteht.

11. Verfahren nach einem der Ansprüche 1 bis 10, bei dem das Retentat aus der Proteinabtrennung (7) in die säurehaltige Lösung vor die Zellabtrennung (4) zurückgeführt wird oder mit vollentsalztem Wasser ausgewaschen wird und die so eluierte Citronensäure einem Ionenaustauscher, einer Membran-Elektrodialyse oder einer Fällung als Calciumsalz zugeführt wird und anschließend in die säurehalige Lösung vor die Zellabtrennung (4) zurückgeführt oder einer weiteren Verwertung zugeführt wird.

12. Verfahren nach einem der Ansprüche 1 bis 11, bei dem das Permeat aus der Proteinabtrennung (7) einem Ionenaustauscher (8) und/oder einer Entfärbung (9) zugeführt wird.

13. Verfahren nach Anspruch 12, bei dem der Ionenaustauscher (8) aus einem stark sauren Kationenaustauscher und/oder einem schwach basischen Anionenaustauscher besteht und bei dem vom Anionenaustauscher mit einer 5 bis 10%-igen Salzsäure-Lösung zuerst Citronensäure eluiert wird und anschließend der Anionenaustauscher mit einer 3 bis 7%-igen Natronlauge-Lösung regeneriert wird oder bei dem der Anionenaustauscher mit einer Ammoniak-Lösung regeneriert wird.

14. Verfahren nach einem der Ansprüche 1 bis 13, bei dem die Kristallisation (11) in drei Stufen (11a, 11b, 11c) erfolgt, wobei die Kristallite aus der ersten Kristallisationsstufe (11a) in vollentsalztem Wasser gelöst werden und einer zweiten Kristallisationsstufe (11 b) zugeführt werden, wobei die Mutterlauge aus der zweiten Kristallisationsstufe (11b) der dritten Kristallisationsstufe (11c) zugeführt wird, wobei die Kristallite aus der dritten Kristallisationsstufe (11c) in vollentsalztem Wasser gelöst werden und in die zweite Kristallisationsstufe (11b) zurückgeführt werden und wobei die Mutterlauge der dritten Kristallisationsstufe (11c) einer weiteren Verwertung zugeführt wird.

15. Verfahren nach einem der Ansprüche 1 bis 14, bei dem die Mutterlauge aus der Kristallisation (11) bzw. dritten Kristallisationsstufe (11c) der Säure neutralisiert wird (14) und das Salz der organischen Säure einer ein- oder mehrstufigen Salzkristallisation zugeführt wird.

16. Verfahren nach Anspruch 15, bei dem die Salzkristallisation in drei Stufen (15a, 15b, 15c) erfolgt, wobei die Mutterlauge aus der ersten Salzkristallisationsstufe (15a) einer zweiten Salzkristallisationsstufe (15b) zugeführt wird, wobei die Kristallite der zweiten Salzkristallisationsstufe (15b) mit denen der ersten Salzkristallisationsstufe (15a) vereinigt werden, wobei die vereinigten Kristallite aus der ersten und zweiten Salzkristallisationsstufe (15a und 15b) in vollentsalztem Wasser gelöst und einer dritten Salzkristallisationsstufe (15c) zugeführt werden und wobei die Mutterlauge aus der dritten Salzkristallisationsstufe (15c) mit der Mutterlauge aus der zweiten Salzkristallisationsstufe (15b) vereinigt wird und einer weiteren Verwendung zugeführt wird.

17. Verfahren nach einem der Ansprüche 14 bis 16, bei dem die Mutterlauge aus der dritten Kristallisationsstufe (11c) oder die Mutterlauge aus der Salzkristallisation bzw. die vereinigte Mutterlauge aus der zweiten und dritten Salzkristallisationsstufe (15b und 15c) einem Ionenaustausch (18) und/oder einer Entfärbung (17) zugeführt wird.

18. Verfahren nach einem der Ansprüche 1 bis 17, bei dem die Kristallite aus der Kristallisation (11) bzw. zweiten Kristallisationsstufe (11b) und/oder der Salzkristallisation bzw. dritten Salzkristallisationsstufe (15c) in einer Flüssigkeit suspendiert und anschließend granuliert werden.

19. Verfahren nach einem der Ansprüche 1 bis 18, bei dem die Mutterlauge aus der Kristallisation (11) bzw. ersten Kristallisationsstufe (11a) und/oder der Salzkristallisation bzw. dritten Salzkristallisationsstufe (11c) einem Ionenaustauscher, einer Membran-Elektrodialyse oder einer Fällung als Calciumsalz zugeführt wird und anschließend in das Permeat aus der Zellabtrennung (4) zurückgeführt oder einer weiteren Verwertung zugeführt wird.

20. Verfahren nach einem der Ansprüche 1 bis 19, bei dem als siliciumhaltiges Fällungsmittel eine 25 bis 35 %-ige SiO₂-Lösung mit SiO₂ einer spezifischen Oberfläche größer 100 m²/g verwendet wird.

21. Verwendung des Verfahren nach einem der Ansprüche 1 bis 20 zur Herstellung von Citronensäure.

## Claims

1. A process for the preparation of an organic acid and/or its salts from a solution which has been obtained by fermentation (3), in which a pure, carbohydrate-containing raw material is used for producing the substrate (1), in which the acid-containing solution prepared by means of fermentation (3) is sent for cell separation (4), in which the acid-containing permeate from the cell separation (4) is sent for protein precipitation (6), with a silicon-containing precipitating agent being added thereto at temperatures between 2 and 70°C, in which the resulting solution is set for protein separation (7) and in which the acid-containing permeate from the protein separation (7) is concentrated (10) and then sent for single-stage or multi-stage crystallisation (11) or granulation.

2. A process according to Claim 1, in which pure glucose, sucrose or pure, enzymatically liquefied starch of raffinate stage 1 having a content of higher sugars of less than 5% is used as the pure, carbohydrate-containing raw material for producing the substrate (1).

3. A process according to Claim 1 or 2, in which the pure, carbohydrate-containing raw material for producing the substrate (1) is sent for cation exchange (2) before its fermentation (3).

4. A process according to one of claims 1 to 3, in which the cell separation (4) is performed with the aid of filtration.

5. A process according to Claim 4, in which the cell separation (4) is effected with the aid of microfiltration with a filter of a pore width of 0.1 to 5 µm.

6. A process according to one of Claims 1 to 5, in which the permeate from the cell separation (4) is heated (5) to a temperature of more than 70°C for a period of 0.5 to 10 min. before protein precipitation (6).

7. A process according to one of Claims 1 to 6, in which the sediment from the protein precipitation (6) is returned into the acid-containing solution before the cell separation (4) after a settling time of 30 to 300 min., and in which the supernatant from the protein precipitation (6) is sent for protein separation (7).

8. A process according to one of Claims 1 to 7, in which the protein separation (7) is performed with the aid of membrane filtration.

9. A process according to Claim 8, in which the protein separation (7) is performed with the aid of membrane filtration which consists of a combination of microfiltration with a filter of a pore width of 0.05 to 5 µm and ultrafiltration having a particle exclusion limit to below 5000 Dalton.

10. A process according to Claim 8, in which the protein separation (7) is performed with the aid of membrane filtration which consists of a combination of microfiltration with a filter of a pore width of 0.05 to 5 µm, ultrafiltration having a particle exclusion limit to below 2000 Dalton and a nanofiltration stage having a particle exclusion limit to below 200 Dalton.

11. A process according to one of Claims 1 to 10, in which the retentate from the protein separation (7) is returned into the acid-containing solution before the cell separation (4) or is washed out with demineralised water and the citric acid thus eluted is sent as calcium salt to an ion exchanger, a membrane electrodialysis stage or a precipitation stage and then is returned into the acid-containing solution before the cell separation (4) or is sent for further use.

12. A process according to one of Claims 1 to 11, in which the permeate from the protein separation (7) is fed to an ion exchanger (8) and/or a decolorisation stage (9).

13. A process according to Claim 12, in which the ion exchanger (8) consists of a strongly acidic cation exchanger and/or a weakly basic anion exchanger and in which first citric acid is eluted from the anion exchanger with a 5 to 10% hydrochloric acid solution and then the anion exchanger is regenerated with a 3 to 7% sodium hydroxide solution or in which the anion exchanger is regenerated with an ammonia solution.

14. A process according to one of Claims 1 to 13, in which the crystallisation (11) takes place in three stages (11a, 11b, 11c), the crystallites from the first crystallisation stage (11a) being dissolved in demineralised water and being sent to a second crystallisation stage (11b), the mother lye from the second crystallisation stage (11b) being sent to the third crystallisation stage (11c), the crystallites from the third crystallisation stage (11c) being dissolved in demineralised water and returned to the second crystallisation stage (11b) and the mother lye of the third crystallisation stage (11c) being sent for further use.

15. A process according to one of Claims 1 to 14, in which the mother lye from the crystallisation (11) or third crystallisation stage (11c) of the acid is neutralised (14) and the salt of the organic acid is sent for single-stage or multi-stage salt crystallisation.

16. A process according to Claim 15, in which the salt crystallisation is effected in three stages (15a, 15b, 15c), the mother lye from the first salt crystallisation stage (15a) being sent to a second salt crystallisation stage (15b), the crystallites from the second crystallisation stage (15b) being combined with those of the first salt crystallisation stage (15a), the combined crystallites from the first and second salt crystallisation stage (15a and 15b) being dissolved in demineralised water and being sent to a third salt crystallisation stage (15c), and the mother lye from the third salt crystallisation stage (15c) being combined with the mother lye from the second salt crystallisation stage (15b) and being sent for further use.

17. A process according to one of Claims 14 to 16, in which the mother lye from the third crystallisation stage (llc) or the mother lye from the salt crystallisation or the combined mother lye from the second and third salt crystallisation stages (15b and 15c) is sent for ion exchange (18) and/or decolorisation (17).

18. A process according to one of Claims 1 to 17, in which the crystallites from the crystallisation (11) or second crystallisation stage (11b) and/or the salt crystallisation or third salt crystallisation stage (15c) are suspended in a liquid and then granulated.

19. A process according to one of Claims 1 to 18, in which the mother lye from the crystallisation (11) or first crystallisation stage (11a) and/or from the salt crystallisation or the third salt crystallisation stage (11c) is sent as a calcium salt to an ion exchanger, a membrane electrodialysis stage or precipitation stage and then is returned to the permeate from the cell separation (4) or is sent for further use.

20. A process according to one of Claims 1 to 19, in which a 25 to 35% SiO₂ solution with SiO₂ of a specific surface area of greater than 100 m²/g is used as the silicon-containing precipitating agent.

21. The use of the process according to one of Claims 1 to 20 for the preparation of citric acid.

## Revendications

1. Procédé de préparation d'un acide organique et/ou de son sel dans une solution qui a été obtenue par fermentation (3), qui consiste à utiliser une matière première contenant un hydrate de carbone pour la préparation (1) du substrat, à envoyer à une séparation (4) de cellule la solution acide préparée au moyen de la fermentation (3), à envoyer le perméat acide de la séparation (4) de cellule à une précipitation (6) de protéine, où il est mélangé à un agent de précipitation contenant du silicium à des températures comprises entre 2 et 70°C, à envoyer la solution ainsi obtenue à une séparation (7) de protéine et à concentrer (10) le perméat acide de la séparation (7) de protéine et ensuite à l'envoyer à une cristallisation (11) à un étage ou à plusieurs étages ou à une granulation.

2. Procédé suivant la revendication 1, qui consiste à utiliser comme matière première pure d'hydrate contenant un hydrate de carbone pour la préparation (1) du substrat, du glucose pur, du saccharose ou de l'amidon pur liquéfié par voie enzymatique du stade 1 de raffinat ayant une proportion en sucres supérieurs inférieure à 5 %.

3. Procédé suivant la revendication 1 ou 2, qui consiste à envoyer la matière première pure contenant de l'hydrate de carbone pour la fabrication (1) du substrat avant sa fermentation (3) à un échange (2) de cations.

4. Procédé suivant l'une des revendications 1 à 3, qui consiste à effectuer la séparation (4) de cellules à l'aide d'une filtration.

5. Procédé suivant la revendication 4, qui consiste à effectuer la séparation (4) de cellules à l'aide d'une micro-filtration avec un filtre d'une dimension de pore de 0,1 à 5 µm.

6. Procédé suivant l'une des revendications 1 à 5, qui consiste à chauffer (5) le perméat provenant de la séparation (4) de cellules avant la précipitation (6) de protéine pendant une durée de 0,5 à 10 minutes à une température supérieure à 70°C.

7. Procédé suivant l'une des revendications 1 à 6, qui consiste à retourner le sédiment provenant de la précipitation (6) de protéine après une durée de décantation de 30 à 300 minutes à la solution acide avant la séparation (4) de cellules, et à envoyer le surnageant de la précipitation (6) de protéine à la séparation (7) de protéines.

8. Procédé suivant l'une des revendications 1 à 7, qui consiste à effectuer la séparation (7) de protéines à l'aide d'une filtration sur membrane.

9. Procédé suivant la revendication 8, qui consiste à effectuer la séparation (7) de protéines à l'aide d'une filtration sur membrane, qui est constituée d'une combinaison d'une micro-filtration avec un filtre ayant une dimension de pore de 0,05 à 5 µm et d'une ultra-filtration ayant une limite d'exclusion des particules allant jusqu'à une valeur inférieure à 5 000 daltons.

10. Procédé suivant la revendication 8, qui consiste à effectuer la séparation (7) de protéines à l'aide d'une filtration sur membrane, qui est constituée d'une combinaison d'une micro-filtration avec un filtre ayant une dimension de pore de 0,05 à 5 µm, d'une ultra-filtration ayant une limite d'exclusion de particules allant jusqu'à une valeur inférieure à 2 000 daltons et d'une nano-filtration ayant une limite d'exclusion de particules allant jusqu'à une valeur inférieure à 200 daltons.

11. Procédé suivant l'une des revendications 1 à 10, qui consiste à retourner le rétentat de la séparation (7) de protéines à la solution acide avant la séparation (4) de cellules ou à lui faire subir un lavage par de l'eau entièrement déminéralisée et à envoyer l'acide citrique ainsi élué à un échangeur d'ions, à une électrodialyse sur membrane ou à une précipitation sous forme de sel de calcium, et -ensuite à le retourner à la solution acide avant la séparation (4) de cellules ou à l'envoyer à une autre valorisation.

12. Procédé suivant l'une des revendications 1 à 11, qui consiste à envoyer le perméat provenant de la séparation (7) de protéines à un échangeur (8) d'ions et/ou à une décoloration (9).

13. Procédé suivant la revendication 12, dans lequel l'échangeur (8) d'ions consiste en un échangeur cationique fortement acide et/ou en un échangeur anionique faiblement basique et dans lequel on élue de l'échangeur anionique par une solution d'acide chlorhydrique à 5 à 10 %, d'abord de l'acide citrique et ensuite on régénère l'échangeur anionique par une solution de liqueur de soude à 3 à 7 %, ou on régénère l'échangeur anionique par une solution d'ammoniaque.

14. Procédé suivant l'une des revendications 1 à 13, qui consiste à effectuer la cristallisation (11) en trois stades (11a, 11b, 11c), les cristallites provenant du premier stade (11a) de cristallisation étant dissous dans de l'eau entièrement déminéralisée et étant envoyés à un deuxième stade (11b) de cristallisation, la liqueur mère du deuxième stade (11b) étant envoyée au troisième stade (11c) de cristallisation, les cristallites provenant du troisième stade (11c) de cristallisation étant dissous dans de l'eau entièrement déminéralisée et étant retournés au deuxième stade (11b) de cristallisation, et la liqueur mère du troisième stade (11c) de cristallisation étant envoyée à une autre valorisation.

15. Procédé suivant l'une des revendications 1 à 14, qui consiste à neutraliser (14) la liqueur mère provenant de la cristallisation (11) ou du troisième stade (11c) de cristallisation de l'acide et à envoyer le sel de l'acide organique à une cristallisation de sel à un stade ou à plusieurs stades.

16. Procédé suivant la revendications 15, qui consiste à effectuer la cristallisation de sel en trois stades (15a, 15b, 15c), la liqueur mère du premier stade (15a) de cristallisation de sel étant envoyée à un deuxième stade (15b) de cristallisation de sel, les cristallites du deuxième stade (15b) de cristallisation de sel étant réunis à ceux du premier stade (15a) de cristallisation de sel, les cristallites réunis provenant du premier et du deuxième stades (15a et 15b) de cristallisation de sel étant dissous dans de l'eau entièrement déminéralisée et étant envoyés à un troisième stade (15c) de cristallisation de sel, et la liqueur mère provenant du troisième stade (15c) de cristallisation de sel étant réunie à la liqueur mère provenant du deuxième stade (15b) de cristallisation de sel et étant envoyée à une autre utilisation.

17. Procédé suivant l'une des revendications 14 à 16, qui consiste à envoyer la liqueur mère provenant du troisième stade (11c) de cristallisation ou la liqueur mère provenant de la cristallisation de sel ou les liqueurs mères réunies provenant du deuxième et du troisième stades (15b, 15c) de cristallisation de sel à un échange (18) d'ions et/ou à une décoloration (17).

18. Procédé suivant l'une des revendications 1 à 17, qui consiste à mettre en suspension dans un liquide des cristallites provenant de la cristallisation (11) ou du deuxième stade (11c) de cristallisation et/ou de la cristallisation de sel ou du troisième stade (15c) de cristallisation de sel et ensuite à les granuler.

19. Procédé suivant l'une des revendications 1 à 18, qui consiste à envoyer la liqueur mère provenant de la cristallisation (11) ou du premier stade (11a) de cristallisation et/ou de la cristallisation de sel ou du troisième stade (11c) de cristallisation de sel à un échangeur d'ions, à une électrodialyse sur membrane ou à une précipitation sous forme de sel de calcium, et ensuite à la retourner dans le perméat provenant de la séparation (4) de cellules ou à une autre valorisation.

20. Procédé suivant l'une des revendications 1 à 19, qui consiste à utiliser comme agent de précipitation contenant du silicium une solution à 25 à 35 % de SiO₂ ayant du SiO₂ d'une surface spécifique supérieure à 100 m²/g.

21. Utilisation du procédé selon l'une des revendications 1 à 20 pour la préparation de l'acide citrique.
